(19) Europäisches Patentamt
European Patent Office
Office européen des brevets

(11) **EP 4 752 753 A1**

(12) **EUROPEAN PATENT APPLICATION**

(43) Date of publication:
03.06.2026 Bulletin 2026/23

(21) Application number: 24215633.9

(22) Date of filing: 27.11.2024

(51) International Patent Classification (IPC):
**G06F 16/9032** (2019.01)  **G16H 40/40** (2018.01)
**G06N 3/091** (2023.01)  **G16H 10/20** (2018.01)
**G16H 50/20** (2018.01)

(52) Cooperative Patent Classification (CPC):
**G06F 16/90332; G06N 3/044; G06N 3/08;
G06N 3/091; G06N 5/01; G06N 5/022; G06N 5/025;
G06N 5/04; G06N 5/041; G06N 5/045; G06N 7/01;
G06N 20/00; G16H 10/20; G16H 40/40;
G16H 50/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: BIOTRONIK SE & Co. KG
12359 Berlin (DE)

(72) Inventors:
• **Wunderlich, Hanno**
**13555 Berlin (DE)**
• **Koeppel, Juergen**
**85599 Parsdorf (DE)**
• **Elsner, Joachim**
**14059 Berlin (DE)**

(74) Representative: **Biotronik Corporate Services SE
Corporate Intellectual Property
Sieversufer 9
12359 Berlin (DE)**

(54) **OPTIMIZATION OF ACTIVE IMPLANT OPERATION PARAMETERS BY AI**

(57) The present invention relates to a computer-implemented method (100) for optimized processing of a question, preferably a medical question. The computer-implemented method (100) comprises selectively obtaining (110), based on the question to be processed, a set of data from a database and processing (120), based on an artificial intelligence (AI), preferably a large language model (LLM), the question. In particular, processing (120) the question is based on the question and the set of data selectively obtained from the database and the processing (120) of the question comprises associating a first answer to the question.

FIG. 1

## Description

Described herein are a method, a system and a computer program for an optimized processing of a question, such as a medical question. The question is processed based on an artificial intelligence.

Improving and optimizing a health state of a patient is at the very heart of advanced medicine and substantial progress has been made in the recent years. Specifically, the impact of novel physiological parameters of the patient on the patient's health state has been revealed and complex correlations between these novel physiological parameters have been uncovered. Tuning these physiological parameters such as to optimize the health of the patient is thus an important issue.

However, due to the increasing complexity and the large scope of the field, maintaining an overview over all the specific details is even for medical professionals, such as general practitioners, almost impossible. Therefore, medical professionals typically pose their questions relating to a particular medical field and/or to a particular medical topic to a person specialized in that field and/or topic, e.g., by sending a message with the question to the specialist. However, as manually answering these questions is a time-consuming process and as the number of questions is growing constantly, this strategy is reaching limits.

Therefore, there is a need for an improved and more efficient processing and answering of medical and other questions, addressing at least some of the above-described disadvantages of the prior art and further improving other aspects.

A first aspect of the relates to a computer-implemented method for optimized processing of a question, preferably a medical question. The computer-implemented method comprises selectively obtaining, based on the question to be processed, a set of data from a database. The computer-implemented method further comprises processing, based on an artificial intelligence (AI) the question, wherein the processing of the question comprises associating a first answer to the question. For example, the AI may comprise a large language model (LLM). In particular, processing the question is based on the question and the set of data selectively obtained from the database.

Selectively obtaining the set of data from a database based on the question to be processed may comprise that the obtained set of data has been selected and/or chosen for the particular question among the data stored in the database. For example, selectively obtaining the set of data may comprise that for a first question to be processed a first set of data is selectively obtained and that for a second question to be processed a second set of data is selectively obtained. In particular, when the first question differs from the second question, e.g., when a subject matter and/or a topic of the first question differs from a subject matter and/or a topic of the second question, the first set of data may differ from the second set of data.

Specifically, selectively obtaining the set of data from the database may be such that the obtained set of data comprises information relating to the question to be processed. For example, selectively obtaining the set of data from the database may comprise that the obtained set of data relates to a topic and/or a field and/or a subject matter associated with the question to be processed. In particular, selectively obtaining the set of data may be at least partially based on an analysis and/or a classification of the question. For example, the question, e.g., a sequence of words, may comprise a word indicative for a specific topic and/or field and/or subject matter. In some embodiments, selectively obtaining the set of data may comprise that a size of the obtained set of data is smaller than a predefined threshold. For example, the predefined threshold may comprise that a storage space associated with the obtained data is smaller than a threshold. In some examples, suitable data may be obtained up until the threshold.

Processing the question based on an artificial intelligence may comprise that the question is processed based on an artificial neural network, and/or based on deep learning and/or based on a generative pre-trained transformer. For example, the artificial intelligence may comprise an artificial intelligence based on supervised learning and/or reinforcement learning. In particular, the artificial intelligence may comprise an artificial intelligence based on reinforcement learning from human feedback.

Processing the question may comprise that a first answer is associated to the question, wherein associating the first answer is based on the question and the obtained set of data. In other words, the obtained set of data may serve as a question-specific set of data and/or question-specific information based on which a first answer is associated by the artificial intelligence to the question. For example, the first answer may be associated to the question based on the information associated with the obtained set of data. In particular, the artificial intelligence may process the question such as to associate and/or generate the first answer to the question based on, e.g., using, the information associated with the question-specific set of data.

Processing the question based on the obtained question-specific, e.g., filtered, set of data allows for processing the question based on a substantially reduced set of input data. For example, instead of processing the question based on a large generic set of data, e.g., a large set of data used for any question to be processed, only a subset of data among the large generic set of data is used for the processing which is specific and/or indicative for the question. Thereby, processing the question based on the obtained question-specific set of data leads to the technical effect of substantially reducing the computation cost and/or the computation time that the artificial intelligence needs for associating the answer to the question. Specifically, as only the smaller question-specific set of data needs to be processed by the artificial intelligence, the computational cost and/or time is minimized and the efficiency of the processing of the question is optimized (for the specific question). In addition, processing the question based on the obtained question-specific set of data enhances the quality and/or the relevance of the associated first answer.

Generally, the question may be associated with a target confidence level for an answer to be associated with the question. In addition, or alternatively, associating the first answer to the question may comprise associating the first answer to the question with a first confidence level.

The target confidence level for the answer to be associated with the question may comprise a confidence level that an answer to be associated with the question should have. In other words, the target confidence level may comprise a condition when an output of the artificial intelligence can be regarded as an answer to the question to be processed. For example, the target confidence level may comprise at least one probability, e.g., a probability that the answer associated with the question correctly addresses the question.

Associating the question with a target confidence level enhances the quality of the answer associated with this question and ensures that the associated answer fulfils a requested (minimal) requirement. In particular, associating the question with a target confidence level contributes to an optimal reliability of the associated answer. An optimal reliability of the associated answer may be of particular importance when the question comprises a medical question.

Similarly, the first answer may be associated with a first confidence level. The first confidence level may comprise an indication of a quality of the first answer, e.g., how well the first answer addresses the question. Specifically, the target confidence level and the first confidence level may comprise a same type of confidence, e.g., the target confidence level and the first confidence level may be comparable. For example, the first confidence level may comprise at least one probability, e.g., a probability that the first answer correctly addresses the question.

Associating the first answer with a first confidence level allows for monitoring and/or assessing the quality of the first answer. Monitoring and/or assessing the quality of the first answer contributes to an optimal reliability of the answer associated with the question. Specifically, the association of the question with a target confidence level and the association of the first answer with a first confidence level leads to the synergistic technical effect of enabling a benchmarking of a (actual) quality of the first answer against the requested quality that the associated answer should have.

Specifically, the computer-implemented method may further comprise extracting a topic from the question. In addition, the computer-implemented method may further comprise determining the target confidence level based on the topic.

Extracting a topic from the question may comprise analysing and/or processing the question. For example, extracting a topic from the question may be at least in part based on natural language processing. In addition, or alternatively, extracting a topic from the question may be based on an identification of a word within the question indicative of a topic of the question. For example, the question may comprise a word indicating that the question comprises a medical question, e.g., relates to a medical topic. In particular, the question may comprise a word indicating that the question relates to active implants, e.g., to cardiac implants. In addition, or alternatively, the question may comprise a word indicating that the question comprises a technical and/or design and/or legal question. In some embodiments, selectively obtaining the set of data may comprise obtaining the topic and/or a field of the question.

In addition, the target confidence level may be determined based on the topic of the question. For example, when a first question comprises a first topic, a first target confidence level may be determined for the first question. In addition, when a second question comprises a second topic, a second target confidence level may be determined for the second question. For example, a target confidence level determined for a medical question may be larger than a target confidence level determined for a technical and/or design question. In particular, a target confidence level determined for a question relating to active implants, e.g., cardiac implants, may be larger than a target confidence level determined for a technical and/or design question.

Determining a target confidence level for the question based on a (extracted) topic of the question allows for a selective and question-specific assignment of the target confidence level to the question. A selective and question-specific assignment of the target confidence level to the question ensures that answers to sensitive questions, e.g., medical questions, address the question with high probability and that answers to generic questions, e.g., technical questions, address the question with a lower probability. Thus, the efficiency of the processing is optimized as only specific questions, e.g., medical questions, need to be answered with a high confidence level.

In general, the computer-implemented method may further comprise outputting the first answer when the first confidence level exceeds the target confidence level.

Outputting the first answer may comprise outputting the first answer to an interface, e.g., a user interface. For example, the answer may be outputted to an agent that has posed the question, e.g., the question to be processed. Outputting the first answer when the first confidence level exceeds the target confidence level may comprise comparing the first confidence level and the target confidence level. In some embodiments, outputting the first answer when the first confidence level exceeds the target confidence level may comprise that the first answer is not outputted when the first confidence level does not exceed the target confidence level.

Outputting the first answer when the first confidence level exceeds the target confidence level ensures that the answer that the agent and/or client obtains addresses the question with a sufficiently high probability. Therefore, the reliability of the outputted answer to the question can be ensured.

In addition, or alternatively, the computer-implemented method may further comprise associating the first confidence level to the first answer by the AI, preferably the LLM.

For example, the first confidence level may be associated to the first answer by the artificial intelligence based on the processing of the question by the artificial intelligence. Specifically, the artificial intelligence may associate the first confidence level to the first answer based on an uncertainty of the first answer. For example, the uncertainty of the first answer may be based on a probability distribution based on which the artificial intelligence processed the question. In particular, the association of the first confidence level may be at least partially based on the obtained set of data.

Associating the first confidence level to the first answer by the artificial intelligence ensures that the first confidence level reflects the uncertainty of the first answer, thereby enabling an assessment of the reliability of the first answer.

Generally, the computer-implemented method may further comprise outputting a message based on the question to a user interface, preferably a graphical user interface (GUI), when the first confidence level is smaller than the target confidence level. In addition, the computer-implemented method may further comprise obtaining, via the user interface, feedback associated with the message, wherein the feedback comprises a second answer.

For example, when the first confidence level associated with the first question is smaller than the target confidence level, e.g., does not exceed the target confidence level, a message based on the question may be outputted to a user interface. A message based on the question may comprise the question. In addition, or alternatively, the message based on the question may comprise the question in a reformulated version. The user interface may comprise a graphical user interface.

Specifically, the user interface, e.g., the graphical user interface, may be such that the message based on the question can be accessed by a client and/or a user. For example, the client and/or the user may comprise a potential (human) trainer for the question. Generally, the user interface may be associated with a specific client and/or user. For example, a first user interface may be associated with a first client and/or user and a second user interface may be associated with a second client and/or user. In particular, each user interface and/or each client and/or user may be associated with a particular expertise. Specifically, outputting the message to a user interface may comprise selecting at least one user interface among a plurality of user interfaces, e.g., a plurality of user interfaces associated with a plurality of (potential) clients and/or users. In particular, selecting the at least one user interface among the plurality of user interfaces may be at least partially based on a topic and/or field associated with the question. For example, the at least one user interface may be selected such that the topic and/or the field associated with the question relates to the expertise associated with the client and/or user. In some embodiments, when the plurality of clients and/or users comprises a client and/or user whose expertise matches the topic and/or field of the question, the message may be outputted to the user interface associated with the user. In addition, or alternatively, the number of interfaces to which the message is outputted may depend on a conformity of the expertise of the clients and/or users and the topic and/or field of the question. For example, when the topic of the question differs from the expertise of each of the clients and/or users, the message may be outputted to a larger number of user interfaces.

In addition, feedback associated with the message may be obtained via the at least one user interface. For example, the client and/or user associated with the user interface may analyse and/or process the message, e.g., analyse and/or process the question on which the message is based. In particular, the client and/or user may analyse and/or process the message such that the feedback comprises a second answer. The second answer may be based on the analysis and/or the processing of the message, e.g., the question, by the client and/or user. Specifically, the second answer may comprise an answer to the question to be processed. For example, the client and/or user may input the second answer to the user interface.

Outputting the message to a user interface when the first confidence level is smaller than the target confidence level and obtaining feedback comprising a second answer allows for associating an answer to the question although the confidence level associated with the first answer does not exceed the (required) target confidence level. In addition, outputting the message to a user interface associated with a client and/or user whose expertise matches the topic and/or field of the question, ensures that the second answer correctly addresses the question with high probability. Therefore, an answer to the question can be provided even when the data stored in the database is not sufficient to answer the question with the target confidence level.

Specifically, the message may be further based on the first answer associated with the question. In addition, or alternatively, the message may be further based on the first confidence level. In addition, or alternatively, the message may be further based on the data obtained from the database. In addition, or alternatively, the message may be further based on a topic associated with the question.

For example, the message may comprise an indication of the first answer associated with the question. In particular, the first message may comprise the first answer associated with the question. In addition, or alternatively, the message may comprise the first confidence level and/or at least a portion of the set of data obtained from the database. For example, the portion of the obtained data included in the message may be based on a relevance of the obtained set of data. For example, a first element, e.g., a document and/or a file, of the obtained set of data may be more relevant to and/or specific for the question than the second element of the obtained set of data. In particular, the message may comprise the first element of the obtained data but not the second element of the obtained set of data. In addition, or alternatively, the message may comprise the topic of the question. For example, the message may comprise the topic of the question at a beginning and/or a title and/or a header of the message. Specifically, arranging the topic of the question at the beginning and/or the title and/or the header of the message may allow the client and/or user to directly evaluate whether the topic of the question matches the expertise of the client and/or user.

A message based on the first answer and/or the first confidence level and/or at least a portion of the obtained set of data and/or a topic facilitates the analysis and/or the processing of the message by the client and/or user. Thereby, the client and/or user may analyse and/or process the message faster such that a second answer to the question can be obtained faster.

In some embodiments, the second answer may be associated with a second confidence level. In addition, the computer-implemented method may further comprise outputting the second answer when the second confidence level exceeds the target confidence level.

The second answer comprised in the feedback may be associated with a second confidence level. For example, the second confidence level may be at least partially based on the expertise associated with the user interface, e.g., the client and/or user of the user interface. Specifically, the second confidence level may be based on a relevance of the client and/or user for the question to be processed. For example, a client with an expertise in a topic and/or field remote from the topic and/or field of the question may be assigned a low relevance. In addition, or alternatively, the second confidence level may be at least partially based on a subjective confidence level associated with the client and/or user. For example, the subjective confidence level associated with the client and/or user may be assigned to the second answer by the client and/or user. For example, the subjective confidence level may be at least partially based on a confidence of the client and/or user that the second answer (correctly) addresses the question.

In addition, or alternatively the second confidence level may be at least partially based on a time at which the second answer has been obtained. For example, the second confidence level may decrease in time starting from the time at which the second answer has been obtained. In particular, the second confidence level may decrease based on an exponential decay. In addition, or alternatively, the second confidence level may be at least partially based on a decay constant assigned to the question. For example, the decay constant may comprise a constant based on a (expected) period of validity of an answer to the question. For example, a question whose answer is (expected to be) based on current and/or up-to-date information may be associated with a decay constant larger than a decay constant associated to a question whose answer is (expected to be) independent of current and/or up-to-date information.

Associating the second answer with a second confidence level, e.g., a second confidence level based on a relevance and/or a subjective confidence of the client, allows to assign a confidence level to the second answer reflecting the (actual) quality of the second answer. Thereby, the quality of the answer to the question is improved and the possibility of providing the agent and/or client with a wrong and/or unrelated answer is minimized.

When the second confidence level exceeds the target confidence level, the second answer may be outputted, e.g., outputted to an agent and/or client that posed the question. For example, the second answer may be outputted to a user interface associated with the agent and/or client. For example, the second answer may be outputted to the agent and/or client together with the question and/or the second confidence level. In some embodiments, outputting the second answer to the agent and/or client may comprise processing the second answer, e.g., processing the second answer by an artificial intelligence. For example, the second answer may be processed by a large language model. For example, processing the second answer may comprise to process the second answer such that processed second answer comprises a pre-defined format.

Outputting the second answer when the second confidence level exceeds the target confidence level allows for providing the agent and/or client with high-quality answers, e.g., answers that are likely to address the question. Thereby, the possibility of providing the answer with a wrong and/or unrelated answer is minimized.

Generally, the computer-implemented method may further comprise storing the question with the associated first answer in the database, when a confidence level associated with the first answer exceeds the target confidence level of the question. In addition, or alternatively, the computer-implemented method may further comprise storing the question with the associated second answer in the database, when a confidence level associated with the second answer exceeds the target confidence level of the question.

For example, when the first answer exceeds the target confidence level, the first answer may be outputted and stored in the database. In particular, the first answer may be stored in the database together with the question to which the first answer is associated. For example, the first answer may be stored in the database as a question-answer pair. In some embodiments, when the first answer to the question has been outputted and the question with the associated first answer has been stored in the database the method for optimized processing of the question may terminate (for the question).

In addition, or alternatively, when the second answer exceeds the target confidence level, the second answer may be outputted and stored in the database. In particular, the second answer may be stored in the database together with the question to which the second answer is associated. For example, the second answer may be stored in the database as a question-answer pair.

Storing the first and/or second answer in the database when the first and/or second confidence level exceeds the target confidence level allows for processing subsequent questions based on the stored first and/or second answer. In particular, when a similar and/or related subsequent question is posed, the first and/or second answer may be selectively obtained from the database and may be comprised in the obtained set of data. Thereby, the processing of the question is optimized as the relevant data for processing the question can already be found in the database, thus reducing the computational cost and/or computational time of the artificial intelligence.

In addition, storing the first and/or second answer in the database when the first and/or second confidence level exceeds the target confidence level leads to the technical effect of enabling an adaptive expansion of the data comprised in the database. In particular, based on the feedback loop, e.g., the outputting of the message and the obtaining of the feedback, additional data, e.g., a question-answer pair, may be stored in the database.

In general, the question may further comprise at least one physiological quantity associated with a patient. In addition, or alternatively, the question may further comprise at least one operation parameter associated with an active implant of the patient.

For example, the question may be at least partially based on the at least one physiological quantity associated with the patient and/or the at least one operation parameter associated with the active implant of the patient. Specifically, the question may comprise a question directed to a correlation between the at least one physiological quantity and the at least one operation parameter associated with the active implant of the patient. For example, the at least one physiological parameter associated with the patient may comprise one or more cardiac parameters and/or cardiac data associated with the patient. In particular, the at least one physiological parameter may comprise at least a portion of an electrocardiogram of the patient. For example, the at least a portion of an electrocardiogram of the patient may comprise data indicative of a width of a QRS complex and/or a morphology of the QRS complex, e.g., adapted for a detection of a left bundle branch block. In addition, or alternatively, the at least a portion of an electrocardiogram of the patient may comprise data adapted for a detection of a trioventricular block, e.g., a trioventricular block of degree two or three. In addition, or alternatively, the at least a portion of an electrocardiogram of the patient may comprise data indicative of an R-R interval and/or a heart rate. In addition, or alternatively, the at least a portion of an electrocardiogram of the patient may comprise data adapted for a detection of a ventricular tachycardia, e.g., the detection of an elongated ventricular tachycardia. Generally, the at least one physiological parameter associated with the patient may further comprise patient-related data, e.g., an age of the patient and/or a weight of the patient and/or at least one medical diagnosis associated with the patient.

Specifically, the active implant may comprise an implantable pulse generator (IPG) and/or an implantable cardioverter-defibrillator (ICD) and/or an implantable cardiac resynchronization therapy defibrillator (CRT-D) and/or an implantable cardiac resynchronization therapy pacemaker (CRT-P) and/or an implantation of a left bundle branch area pacing implant (LBBAP).

In addition, or alternatively, the at least one operation parameter associated with an active implant of the patient may comprise a recovery time associated with the active implant, e.g., a time between consecutive pacing stimuli. In addition, or alternatively, the at least one operation parameter associated with an active implant of the patient may comprise a capture threshold, e.g., at least one parameter associated with a pacing voltage and/or pacing pulse width.

Processing a question comprising at least one physiologic parameter of the patient and at least one operation parameter associated with an implant of the patient allows for associating the first and/or second answer to the question based on information associated with an (actual) state of the patient and/or an (actual) configuration of the active implant. Therefore, the quality of the first and/or second answer may be significantly improved, particularly when the question is directed to a correlation between the at least one physiological quantity and the at least one operation parameter associated with the active implant of the patient.

Specifically, the at least one physiological quantity associated with a patient may be measured by the active implant of the patient. In addition, or alternatively, the answer may comprise an indication of whether the at least one operation parameter is in accordance with the at least one physiological quantity. In addition, or alternatively, the answer may comprise an indication of at least one updated operation parameter for the active implant.

For example, the at least one physiological parameter may be measured by the active implant to which the at least one operation parameter is associated. In other words, the implant operating based on the at least one operation parameter may measure the at least one physiological parameter. Specifically, the implant may comprise an IPG, ICD, CRT-D, CRT-P and/or LBBAP.

In addition, when the question is directed to a correlation between the (measured) at least one physiological quantity and the at least one operation parameter associated with the active implant of the patient, the answer may comprise an indication of whether the at least one operation parameter is in accordance with the at least one physiological parameter. For example, the indication may indicate that the at least one operation parameter is in accordance with the at least one physiological parameter, e.g., the operation parameters comprise a sensible and/or correct choice of the operation parameters given the health state, e.g., the at least one physiological parameter, of the patient.

In addition, or alternatively, the answer may comprise an indication of at least one updated operation parameter for the active implant, e.g., when at least one of the operation parameters is not in accordance with the at least one physiological quantity. For example, the indication of the at least one updated operation parameter may be outputted to the agent and/or client, e.g., a medical health care provider and/or a physician.

In addition, or alternatively, the at least one updated operation parameter may be outputted to the active implant, e.g., the active implant to which the updated operation parameters are associated. For example, outputting the at least one updated operation parameter to the active implant may comprise transmitting the at least one updated operation parameter to the implant. In some embodiments, the implant may adapt the (current) operation parameters based on the at least one updated operation parameter. For example, the implant may adjust the (current) operation parameters such as to align them to with the at least one updated operation parameter.

In addition, or alternatively, the answer may be outputted to the agent and/or client and to the implant. For example, upon receiving the answer, the implant may store the answer in a memory until the agent and/or client reviews the answer. Specifically, when the agent and/or client approves the answer, e.g., the at least one updated operation parameter, the implant may adjust the (current) operation parameters such as to align them to with the at least one updated operation parameter. In addition, or alternatively, when the agent and/or client rejects the answer, e.g., the at least one updated operation parameter, the implant may delete the answer from the memory.

An answer comprising an indication of whether the at least one physiological parameter is in accordance with the at least one operation parameter of the implant allows for a validation of the current operation parameters of the patient. In addition, an answer comprising an indication of at least one updated operation parameter enables the adjustment of the operation parameters based on the (actual) health state of patient. Thereby, the operation of the active implant of the patient is optimized and can be adjusted to the (actual) health state of the patient.

Generally, the database may comprise questions with an associated answer. In addition, or alternatively, the database may comprise medical documents. For example, the database may comprise medical guidelines. In addition, or alternatively, the database may comprise physiological parameters associated with a patient. In addition, or alternatively, the database may comprise operation parameters associated with an implant of a patient.

For example, the database may comprise (already) posed questions with the associated first and/or second answer. In addition, or alternatively, the database may comprise medical guidelines and/or medical regulations. In addition, or alternatively, the database may comprise physiological parameters of a patient. For example, the at least one physiological parameter of the patient may comprise an identification of the patient, e.g., an indication relating the patient with the physiological parameter.

Specifically, the question with the associated answer may comprise an indication of a time when the answer has been associated with the question. In addition, or alternatively, the at least one physiological parameter associated with a patient may comprise an indication of a time when the at least one physiological parameter has been measured. In addition, or alternatively, at least one operational parameter may comprise an indication of a time when the at least one operational parameter has been adjusted.

In some embodiments, the database comprises a vector database. Specifically, obtaining the set of data from the database may comprise vectorizing the question based on a predefined vector mapping. In addition, obtaining the set of data from the database may comprise obtaining the data from the database based on an optimization of a cost function. For example, obtaining the data from the database based on an optimization of a cost function may comprise a minimization of a distance function.

For example, vectorizing the question to be processed may comprise assigning a vector to the question. In particular, assigning a vector to the question may be based on a predefined vector mapping, e.g., a vector embedding. In addition, selectively obtaining the set of data from the database may be based on an optimization of a cost function. For example, the optimization of the cost function may be based on (or with respect to) the vector assigned to the question. In particular, selectively obtaining the set of data from the database may comprise to optimize the cost function over at least a portion of data stored in the database, e.g., evaluating the cost function for all data elements and/or data files within the at least portion of the data stored in the database. Specifically, selectively obtaining the set of data from the database based on the optimization may comprise that the obtained set of data optimizes the cost function, e.g., the data elements and/or data files in the set of data may optimize the cost function. In some embodiments, the cost function may comprise a distance function, e.g., a function quantifying a distance between two vectors of a vector space.

Selectively obtaining the set of data from the database based on the optimization of a cost function allows for obtaining relevant and related data elements and/or data files from the database based on which the artificial intelligence processes the question. In particular, the performance of the processing and/or the quality of the (first) answer associated with the question depends on the quality of the pre-selected, e.g., selectively obtained set of data. In addition, the evaluation of a distance function between at least two vectors is numerically cheap, e.g., has a low computational cost and a low computation time. Therefore, obtaining the set of data from the database based on the optimization of a cost function minimizes the optimization time, maximizes the quality and the significance of the obtained set of data and thereby optimizes the processing of the question.

A second aspect relates to a system comprising at least one processing unit. The at least one processing unit is configured to perform the computer-implemented method described herein.

A third aspect relates to a computer program comprising instructions which, when the program is executed by a computer, cause the computer to carry out the methods described herein.

Whether aspects are implemented as hardware or software means depends upon the particular application and design constraints imposed on the overall system. By way of example, an element, or any portion of an element, or any combination of elements may be implemented as a processing system that may include one or more processors. Examples of processors include microprocessors, microcontrollers, graphics processing units (GPUs), central processing units (CPUs), application processors, digital signal processors (DSPs), reduced instruction set computing (RISC) processors, systems on a chip (SoC), baseband processors, field programmable gate arrays (FPGAs), programmable logic devices (PLDs), state machines, gated logic, discrete hardware circuits, and other suitable hardware configured to perform the various functionality described throughout this disclosure. One or more processors in the processing system may execute software. Software shall be construed broadly to mean instructions, instruction sets, code, code segments, program code, programs, subprograms, software components, applications, software applications, software packages, routines, subroutines, objects, executables, threads of execution, procedures, functions, etc., whether referred to as software, firmware, middleware, microcode, hardware description language, or otherwise.

Accordingly, in one or more exemplary embodiments, the functions described may be implemented in hardware, software, or any combination thereof. If implemented in software, the functions may be stored on or encoded as one or more instructions or code on a computer-readable medium. Computer-readable media includes computer storage media. Storage media may be any available media that can be accessed by a computer. By way of example, and not limitation, such computer-readable media can comprise a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that can be used to store computer executable code in the form of instructions or data structures that can be accessed by a computer.

It is noted that any combination of features that have been described above as belonging to certain embodiments/aspects of the present invention is also an embodiment of the present invention, provided such a feature combination is feasible, i.e., does not lead to any contradictions.

In the following, exemplary embodiments of the invention are described with reference to the figures. The figures show:

Fig. 1　Schematic illustration of an embodiment of the computer-implemented method according to the present invention;

Fig. 2　Schematic illustration of an embodiment of a system configured to perform the method according to the present invention.

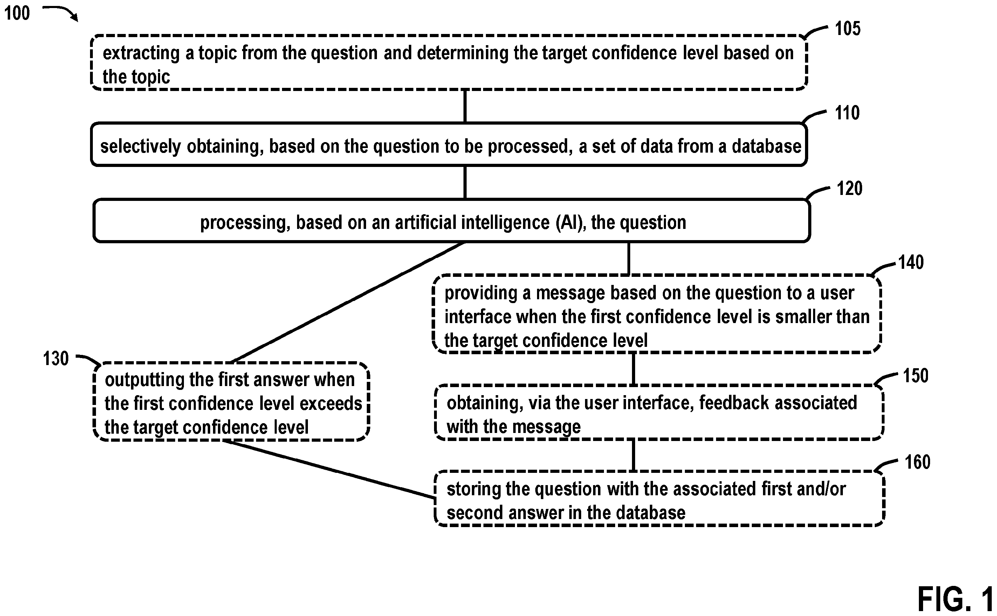

Figure 1 illustrates an exemplary embodiment of a computer implemented method 100 for optimized processing of a question. For example, the method 100 may comprise receiving a question to be processed. The question to be processed may be received via an interface, e.g., via an interface from an agent and/or a client. In addition, or alternatively, the method 100 may further comprise a pre-processing of the question. For example, receiving the question to be processed may comprise receiving a set of questions and/or a questionnaire. Pre-processing the question may comprise separating the questions in the set of questions and/or the questionnaire. Generally, the question may comprise at least one physiological quantity associated with a patient and/or at least one operation parameter associated with an implant, e.g., an active cardiac implant. For example, the at least one physiological quantity associated with the patient may be measured by the active implant of the patient.

The method 100 may further comprise extracting 105 a topic from the question. For example, extracting 105 a topic from the question may comprise extracting that the question relates to backend services associated with an implant and/or to external devices associated with an implant and/or to an implant of a patient, e.g., to a configuration of operation parameters of the implant. In addition, the method 100 may further comprise determining a target confidence level based on the topic. In some embodiments, the target confidence level may be predefined, e.g., the question may comprise a target confidence level. In particular, the agent and/or client posing the question may determine the target confidence level.

In addition, the method 100 may further comprise selectively obtaining 110, based on the question to be processed, a set of data from a data base. For example, the obtained set of data may comprise question-specific data. In particular, when the question relates to a configuration of operation parameters of an implant of a patient, the obtained set of data may comprise patient-specific data. For example, patient specific data may comprise former operation parameter of the implant of the patient and/or former physiological parameters associated with the patient and/or medical diagnostics associated with the patient. Generally, the set of data may be selectively obtained from a vector database. Specifically, selectively obtaining 110 may comprise that the obtained set of data is based on an optimization of a cost function, an optimization of a distance function.

The method 100 may further comprise processing 120, based on an artificial intelligence, the question. For example, the question may be processed based on a large language model. Specifically, processing 120 the question may be based on the question and the set of data selectively obtained from the database. In particular, processing 120 the question may comprise associating a first answer to the question. Generally, processing 120 the question may comprise associating a first confidence level to the first answer. For example, the artificial intelligence, e.g., the large language model, may associate the first confidence level to the first answer.

Specifically, when the question is directed to a correlation between the at least one physiological quantity and the at least one operation parameter associated with the active implant of the patient, the answer may comprise an indication of whether the at least one operation parameter is in accordance with the at least one physiological quantity. In addition, or alternatively, the answer may comprise an indication of at least one updated operation parameter for the implant.

Generally, when the first confidence level associated with the first answer exceeds the target confidence level associated with the question to be processed, the method 100 may further comprise outputting 130 the first answer. For example, the outputting 130 the first answer may comprise outputting the first answer to the agent and/or client, e.g., the health care provider and/or the physician, who has posed the question. In particular, outputting 130 the first question may comprise transmitting the first answer to the agent and/or client who has posed the question.

Generally, the method 100 may further comprise storing 160 the question with the associated first answer in the database. For example, storing 160 the question with the associated first answer in the database may comprise storing the question with the associated first answer when the first confidence level associated with the first answer exceeds the target confidence level.

In some embodiments, when the first confidence level associated with the first answer is smaller than the target confidence level, the method 100 may further comprise outputting 140 a message based on the question to a user interface. Specifically, when the first confidence level associated with the first answer is smaller than the target confidence level, the first answer may not be outputted to the agent and/or to the client. In other words, the step of outputting 130 may not be performed.

Generally, outputting 140 the message to a user interface may comprise outputting the message to at least one user interface. For example, the message may be outputted to a plurality of user interfaces, e.g., the message may be outputted to a first and to a second user interface. Specifically, the message may be based on the question, e.g., the message may comprise the question. In addition, or alternatively, the message may comprise the first answer and/or the first confidence level and/or at least a portion of the obtained set of data.

For example, when the question is directed to a correlation between the at least one physiological quantity and at least one operation parameter associated with an active implant of the patient, the message may comprise the at least one physiological quantity and/or the at least one operation parameter associated with the active implant. In addition, or alternatively, the message may comprise a type of the implant and/or former physiological quantities associated with the patient and/or former operation parameters associated with the implant and/or at least one medical diagnostic associated with the patient.

Each user interface may be associated to a corresponding user, e.g., a trainer for the question. For example, the user may comprise a physician and/or an engineer. In addition, each user may be associated with an expertise, e.g., a field and/or a topic for which the user is competent. For example, a first user may be associated with a first expertise and a second user may be associated with a second expertise. Generally, upon reception of the message, the user may analyse and/or process the question. In particular, the user may process and/or analyse the question such as to associate a second answer to the question. In addition, the user may associate to the second answer a subjective confidence level. Specifically, when the message has been outputted to a plurality of users, the $k^{th}$ user may associate a $k^{th}$ subjective confidence level $\beta_k$ to the respective second answer.

The method 100 may further comprise obtaining 150, via the user interface, feedback associated with the message. The feedback associated with the message may comprise the second answer associated with the message. For example, a first feedback may be obtained from a first user via a first user interface and a second feedback may be obtained from a second user via a second user interface. In particular, the first feedback may comprise a first second answer to the question and a first subjective confidence level and the second feedback may comprise a second second answer to the question and a second subjective confidence level.

Specifically, the method may further comprise processing the obtained second answer of the user, e.g., by the artificial intelligence. For example, when a plurality of second answers has been obtained for the question, e.g., obtained from a plurality of users, the plurality of second answers may be processed by the artificial intelligence, e.g., by the large language model. For example, the artificial intelligence may process the plurality of second answers such as to associate a third answer to the question.

In addition, processing the obtained second answers such as to associate a third answer to the question may comprise associating a third confidence level to the third answer. For example, the third confidence level may be based on a relevance associated with each of the users. In particular, the relevance associated to a user may be based on the question to be processed. For example, the relevance associated to a user may be based on the field and/or topic of the question. Generally, the relevance associated to a user may comprise a measure quantifying whether the expertise of the user matches and/or corresponds to the field and/or topic of the question. For example, for the question to be processed, the kth user may be associated with a $k^{th}$ relevance $\sigma_k$. In addition, or alternatively, the third confidence level may be based on a time when the second answer has been obtained from the user. For example, the second answer of the $k^{th}$ user may have been obtained at a time $t_k$. In addition, or alternatively, the third confidence level may be based on a decay constant $\alpha$ associated with the question to be processed.

Generally, the method 100 may further comprise storing 160 the question with the associated second answer and/or the associated third answer in the database. For example, the decay constant $\alpha$ may comprise a constant based on a (expected) period of validity of an answer to the question. For example, a question whose answer is (expected to be) based on current and/or up-to-date information may be associated with a decay constant larger than a decay constant associated to a question whose answer is (expected to be) independent of current and/or up-to-date information. Specifically, the decay constant $\alpha$ may be at least partially based on the topic associated with the question. In some embodiments, the decay constant $\alpha$ may be assigned to the question based on the artificial intelligence.

In addition, or alternatively, the third confidence level associated with the third answer may comprise a time dependence. For example, the third confidence level may decrease over time, e.g., the third confidence level may decrease based on an exponential decay. Specifically, when feedback of N users has been obtained, the third confidence level may comprise a confidence level $C(t)$ which has been calculated according to

$$C(t) = \frac{1}{S_N} \sum_{k=1} \sigma_k \times \beta_k \times e^{-\alpha(t-t_k)},$$

wherein $S_N$ may comprise a normalization, e.g., $S_N = \Sigma_k{=}_1 \beta_k$.

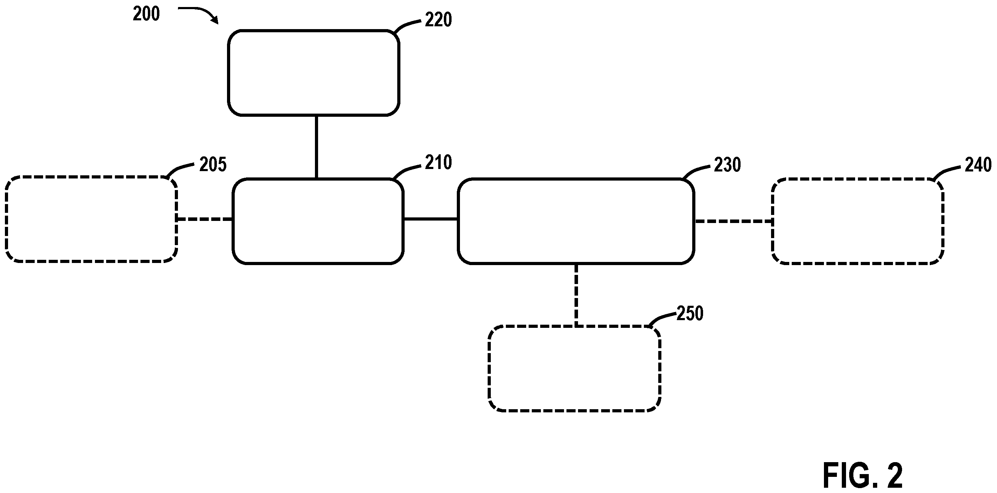

Figure 2 shows a schematic embodiment of a system 200 for performing the computer-implemented method. The system 200 may comprise means for obtaining 205 the question to be processed. For example, the means for obtaining 205 may comprise means for receiving the question to be processed. Specifically, the means for obtaining 205 may comprise an interface. In addition, the system 200 may comprise means for pre-processing the question. For example, the means for pre-processing the question may be adapted to associate a topic and/or a field to the question. In addition, or alternatively, the means for pre-processing may be adapted to separate a question from a set of questions and/or a questionnaire. In addition, the system may further comprise means for selectively obtaining 210, based on the question to be processed, a set of data from a database. In some embodiments, the means for pre-processing the question may be comprised in the means for selectively obtaining 210. The system 200 may further comprise a database 220. For example, the database 220 may comprise questions with an associated answer and/or medical documents, e.g., medical guidelines, and/or physiological parameters associated with a patient and/or operation parameters associated with an implant of the patient. The database 220 may comprise a vector database.

The system 200 may further comprise means for processing 230, based on an artificial intelligence, the question. For example, the means for processing 230 may be adapted such as to process the question based on a large language model. In addition, or alternatively, the means for processing 230 may be adapted to process the question based on the question and the set of data selectively obtained from the database 220. In particular, the means for processing may be adapted such as to associate a first answer to the question.

In addition, or alternatively, the system 200 may comprise means for outputting 240 the first answer. For example, the means for outputting 240 may be configured such as to output the first answer when a confidence level associated with the first answer exceeds a target confidence level associated with the question.

In addition, or alternatively, the system 200 may comprise means for outputting 250 a message based on the question. For example, the means for outputting 250 may be adapted to output the message for sending to a user interface. In some examples, system 200 may comprise the user interface. In addition, the system 200 may further comprise means for obtaining feedback associated with the message. For example, the means for obtaining feedback may be comprised in the means for outputting 250 which may be adapted to obtain the feedback via the user interface.

**Claims**

1. Computer-implemented method (100) for optimized processing of a question, preferably a medical question, comprising:

    selectively obtaining (110), based on the question to be processed, a set of data from a database;
    processing (120), based on an artificial intelligence (AI), preferably a large language model (LLM), the question;
    wherein processing (120) the question is based on the question and the set of data selectively obtained from the database;
    wherein the processing (120) of the question comprises associating a first answer to the question.

2. Computer-implemented method (100) for optimized processing according to claim 1, wherein:

    the question is associated with a target confidence level for an answer to be associated with the question; and/or
    associating the first answer to the question comprises associating the first answer to the question with a first confidence level.

3. Computer-implemented method (100) for optimized processing according to claim 2, wherein the method comprises extracting (105) a topic from the question and determining the target confidence level based on the topic.

4. Computer-implemented method (100) for optimized processing according to claim 2 or 3, wherein the method further comprises outputting (130) the first answer when the first confidence level exceeds the target confidence level.

5. Computer-implemented method (100) for optimized processing according to one of the claims 2 or 4, wherein the method further comprises associating the first confidence level to the first answer by the AI, preferably the LLM.

6. Computer-implemented method (100) for optimized processing according to one of the claims 2 to 5, further comprising:

    providing (140) a message based on the question to a user interface, preferably a graphical user interface (GUI), when the first confidence level is smaller than the target confidence level;

    obtaining (150), via the user interface, feedback associated with the message, wherein the feedback comprises a second answer.

7. Computer-implemented method (100) for optimized processing according to claim 6, wherein the message is further based on the first answer associated with the question and/or the first confidence level and/or the data obtained from the database and/or a topic associated with the question.

8. Computer-implemented method (100) for optimized processing according to claim 6 or 7, wherein the second answer is associated with a second confidence level; preferably further comprising outputting the second answer when the second confidence level exceeds the target confidence level.

9. Computer-implemented method (100) for optimized processing according to one of the claims 1 to 8, further comprising:
    storing (160) the question with the associated first and/or second answer in the database, when a confidence level associated with the first and/or second answer exceeds the target confidence level of the question.

10. Computer-implemented method (100) for optimized processing according to one of the claims 1 to 9, wherein the question further comprises at least one physiological quantity associated with a patient and at least one operation parameter associated with an active implant of the patient.

11. Computer-implemented method (100) for optimized processing according to claim 10, wherein:

    the at least one physiological quantity associated with a patient has been measured by the active implant of the patient; and
    the answer comprises: an indication of whether the at least one operation parameter is in accordance with the at least one physiological quantity, and/or at least one updated operation parameter for the active implant.

12. Computer-implemented method (100) for optimized processing according to one of the claims 1 to 11, wherein the database comprises:

    questions with an associated answer; and/or
    medical documents, preferably medical guidelines; and/or
    physiological parameters of a patient and/or operation parameters associated with an implant of the patient.

13. Computer-implemented method (100) for optimized processing according to one of the claims 1 to 12, wherein the database comprises a vector database and obtaining the set of data from the database comprises:

vectorizing the question to be processed based on a predefined vector mapping; and
obtaining the data from the database based on an optimization of a cost function, preferably based on a minimization of a distance function.

14. A system comprising at least one processing unit configured to perform the method according to one of the claims 1 to 13.

15. A computer program comprising instructions which, when the program is executed by a computer, cause the computer to carry out the method according to one of the claims 1 to 13.

100 ⟍

EP 4 752 753 A1

```
┌─────────────────────────────────────────────────────────────────────────────┐  ⟋ 105
┆ extracting a topic from the question and determining the target confidence level based on  ┆
┆ the topic                                                                     ┆
└─────────────────────────────────────────────────────────────────────────────┘
```

```
┌─────────────────────────────────────────────────────────────────────────────┐  ⟋ 110
│ selectively obtaining, based on the question to be processed, a set of data from a database │
└─────────────────────────────────────────────────────────────────────────────┘
```

```
┌─────────────────────────────────────────────────────────────────────────────┐  ⟋ 120
│                processing, based on an artificial intelligence (AI), the question            │
└─────────────────────────────────────────────────────────────────────────────┘
```

```
                                                    ┌───────────────────────────────────┐  ⟋ 140
                                                    ┆ providing a message based on the question to a user  ┆
                                                    ┆ interface when the first confidence level is smaller than ┆
                                                    ┆ the target confidence level         ┆
                                                    └───────────────────────────────────┘
```

130 ⟍
```
┌───────────────────────────┐              ┌───────────────────────────────────┐  ⟋ 150
┆ outputting the first answer when ┆        ┆ obtaining, via the user interface, feedback associated ┆
┆ the first confidence level exceeds ┆      ┆ with the message                    ┆
┆ the target confidence level   ┆          └───────────────────────────────────┘
└───────────────────────────┘
```

```
                                          ┌───────────────────────────────────┐  ⟋ 160
                                          ┆ storing the question with the associated first and/or ┆
                                          ┆ second answer in the database       ┆
                                          └───────────────────────────────────┘
```

FIG. 1

FIG. 2

| | EUROPEAN SEARCH REPORT | Application Number |
|---|---|---|
| Europäisches Patentamt / European Patent Office / Office européen des brevets | | EP 24 21 5633 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YANG BUFANG ET AL: "DrHouse: An LLM-empowered Diagnostic Reasoning System through Harnessing Outcomes from Sensor Data and Expert Knowledge", PROCEEDINGS OF THE ACM ON INTERACTIVE, MOBILE, WEARABLE AND UBIQUITOUS TECHNOLOGIES, ACMPUB27, NEW YORK, NY, USA, vol. 8, no. 4, 21 November 2024 (2024-11-21), pages 1-29, XP059774261, DOI: 10.1145/3699765 * the whole document * ----- | 1-15 | INV. G06F16/9032 G16H40/40 G06N3/091 G16H10/20 G16H50/20 |
| | | | **TECHNICAL FIELDS SEARCHED (IPC)** |
| | | | G06F G16H G06N |

The present search report has been drawn up for all claims

| Place of search | Date of completion of the search | Examiner |
|---|---|---|
| The Hague | 15 April 2025 | Ntarlagiannis, V |

EPO FORM 1503 03.82 (P04C01)